(19) **Europäisches Patentamt**
**European Patent Office**
**Office européen des brevets**

(11) **EP 3 431 465 A1**

(12) **EUROPEAN PATENT APPLICATION**

(43) Date of publication:
**23.01.2019 Bulletin 2019/04**

(51) Int Cl.:
***C07C 319/20*** (2006.01)   ***C07C 319/28*** (2006.01)
***C07C 323/58*** (2006.01)

(21) Application number: **17182515.1**

(22) Date of filing: **21.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Evonik Degussa GmbH**
**45128 Essen (DE)**

(72) Inventors:
• **BERNHARDT, Sebastian**
  **63739 Aschaffenburg (DE)**
• **KÖRFER, Martin**
  **63796 Kahl (DE)**
• **REICHERT, Stefan**
  **60438 Frankfurt (DE)**
• **HASSELBACH, Hans Joachim**
  **63571 Geinhausen (DE)**
• **DRAPAL, Bernd**
  **63755 Alzenau (DE)**
• **PETER, Rainer**
  **63829 Krombach (DE)**

(54) **PROCESS FOR PREPARING METHIONINE**

(57)    The present invention relates to a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate wherein the alkali metal cations in the process solution are potassium and sodium having a K/Na molar ratio range from 99/1 to 20/80 and to an aqueous fertilizer resulting from this process.

EP 3 431 465 A1

**Description**

The present invention relates to a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate wherein the alkali metal cations in the process solution are potassium and sodium having a K/Na molar ratio range from 99/1 to 20/80.

The amino acid methionine is currently industrially produced worldwide in large amounts and is of considerable commercial importance. Methionine is employed in many fields, such as pharmaceutical, health and fitness products, but particularly as feedstuff additive in many feedstuffs for various livestock.

On an industrial scale, methionine is produced chemically via the Bucherer-Bergs reaction, which is a variant of the Strecker synthesis. Here, the starting substances 3-methylmercaptopropanal (prepared from 2-propenal and methylmercaptan), hydrocyanic acid (hydrogen cyanide), ammonia and carbon dioxide are reacted to give 5-(2-methylmercaptoethyl)hydantoin (methionine hydantoin) and this is subsequently hydrolysed by alkali with potassium carbonate and potassium hydrogen carbonate to give potassium methioninate. Methionine is finally liberated from its potassium salt by treatment with carbon dioxide ("carbonation reaction"), which may be filtered off as a precipitate from the mother liquor containing potassium carbonate and potassium hydrogen carbonate (US 5,770,769). The precipitated methionine is then recrystallized from an aqueous solution, filtered off and dried. The methionine so obtained has a purity greater than 99 % and a potassium content of less than 0.5 %.

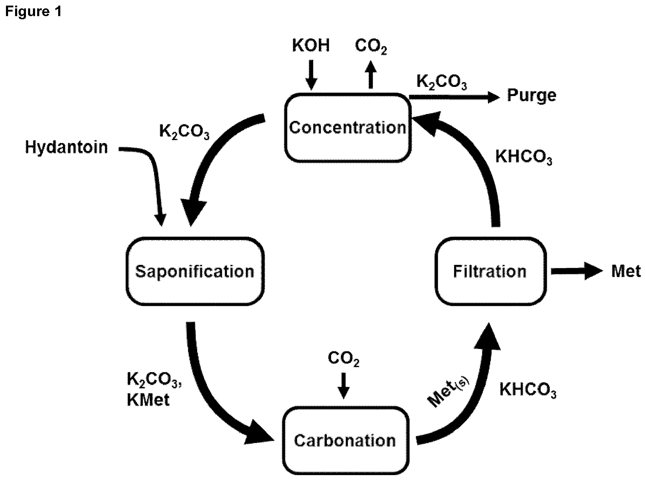

The ammonia, potassium carbonate and potassium hydrogen carbonate reagents and also carbon dioxide are generally recycled in industrial methionine production. However, it is necessary to continuously exchange a part of the aqueous process solution of this hydantoin hydrolysis circulation for fresh potassium hydroxide, essentially to remove ("purge") inactivated potassium salt from the circulation in the form of potassium formate. Potassium formate forms from the residues of hydrogen cyanide being present in the methionine hydantoin solution and alkaline potassium salts from the hydantoin hydrolysis (WO2013030068A2). A further by-product of the methionine synthesis is the dipeptide methionylmethionine (EP 1 564 208 A1). In general, the excessive enrichment of by-products in the hydantoin hydrolysis circulation must be avoided since otherwise disruptions in crystal formation occur downstream. A scheme of the hydantoin hydrolysis circulation is shown in figure **1**.

A typical process solution recycled to the hydantoin hydrolysis circulation comprises approximately 10 to 16 % by weight potassium, 5 to 10 % by weight methionine, 3 to 5 % by weight methionylmethionine, 0.5 to 2.0 % by weight formate, and 0.2 to 0.3 % by weight acetate.

Methionate and potassium may be partially recovered from the purge stream by precipitation with carbon dioxide in form of methionine and potassium carbonate, respectively, filtered off and fed back to the process solution. However, there is still a considerable loss of the relatively expensive potassium salts. The final purge solution comprises approximately 2 to 6 % by weight methionine, 4 to 8 % by weight methionylmethionine and 6 to 14 % by weight potassium in the form of potassium salts, 1 to 1.7 % by weight formate, and 0.3 to 0.5 % by weight acetate.

With the aim to recycle the excess of sodium carbonate Capelle and Rey (WO 2016/170252 A1) propose a method for producing methionine by alkaline hydrolysis of methionine hydantoin in aqueous phase, eliminating ammonia and carbon dioxide from the hydrolysis medium, and neutralising the methionine sodium salt obtained, wherein after the elimination of ammonia and carbon dioxide the reaction medium is concentrated in order to precipitate sodium carbonate which is filtered off and recycled for alkaline hydrolysis in the presence of sodium hydroxide and sodium carbonate. However, in this method the neutralisation of the methionine sodium salt is achieved with sulphurous acid still leading to large amounts of sodium sulphate as side product which has to be removed from the process.

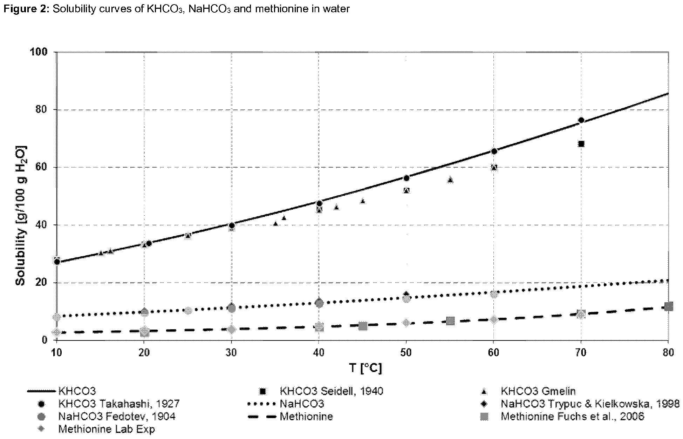

In the carbonation reaction, i.e. reaction of the alkali metal salt of methionine with carbon dioxide to form methionine and alkali metal hydrogencarbonate (Figure 1), methionine is precipitated and removed from the process solution. However, due to the low difference in the solubility of sodium hydrogencarbonate and methionine (Figure 2), large quantities of sodium hydrogencarbonate are co-precipitated with methionine in case that sodium salt of methionine is used in this reaction. Therefore, in the carbonisation reaction the potassium salt of methionine is used, meaning that potassium hydroxide usually is added to the hydantoin hydrolysis circulation process.

The object of the present invention is to provide a process for preparing methionine, comprising the alkaline hydrolysis of 5-(2-methylmercaptoethyl)-hydantoin (methionine hydantoin) in an aqueous process solution comprising potassium hydroxide and/or carbonate and/or hydrogencarbonate to form methionine potassium salt, precipitation of methionine by means of carbon dioxide, separation of the precipitated methionine from the process solution, concentration of the process solution and recycling of the process solution to the alkaline hydrolysis of methionine hydantoin, wherein the potassium cations in the process solution are partly replaced by sodium cations in order to avoid losses of the relatively expensive potassium salts in the purge solution stream.

It was found that in the process solution resulting from the hydantoin hydrolysis reaction a large part of potassium can be replaced by sodium without the undesired co-precipitation of sodium hydrogencarbonate in the carbonation reaction.

This object is achieved by a process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate to form methionine alkali metal salt, precipitation of methionine by means of carbon dioxide, separation of the precipitated methionine from the process solution, concentration of the process solution and recycling of the process solution in the alkaline hydrolysis of methionine hydantoin once more forwarded to the process solution, wherein the alkali metal cations in the process solution are potassium and sodium having a K/Na molar ratio range from 99/1 to 20/80 resulting in potassium concentrations ranging from 13.9 % by weight to 1.2 % by weight and sodium concentrations ranging from 0.04 % by weight to 6.6 % by weight.

During the concentration step the process solution is re-concentrated to its typical starting concentrations of the hydantoin hydrolysis circulation, i.e. comprising approximately 5 to 16 % by weight alkali metal, 5 to 8 % by weight methionine, 3 to 5 % by weight methionylmethionine, 0.7 to 1.1 % by weight formate, and 0.2 to 0.3 % by weight acetate.

In the process according to the present invention the K/Na molar ratio in the process solution preferably ranges from 90/10 to 20/80 resulting in potassium concentrations ranging from 12,6 % by weight to 1,2 % by weight and sodium concentrations ranging from 0,35 % by weight to 6,6 % by weight. Ideally, the K/Na molar ratio in the process solution ranges from 70/30 to 50/50 resulting in potassium concentrations ranging from 9,8 % by weight to 3 % by weight and sodium concentrations ranging from 1,06 % by weight to 4,1 % by weight.

In the process according to the present invention the precipitated methionine is then recrystallized from an aqueous solution, filtered off and dried. The methionine so obtained has a purity greater than 99 % and a potassium content lower than 0.3 % and a sodium content lower than 0.1 %, in particular the potassium content in the crystalline methionine ranges from 0.05 - 0.3 % and the sodium content ranges from 0.01 - 0.1 %.

In the process according to the present invention a part of the process solution, the so called "purge solution", may continuously be removed from the process and exchanged by fresh alkali metal hydroxide solution.

The purge solution removed from the process comprises 2 to 6 % by weight methionine, 4 to 8 % by weight methionylmethionine, 1 to 1.7 % by weight formate, 0.3 to 0.5 % by weight acetate and 6 to 14 % by weight alkali metal in the form of potassium and sodium salts, potassium and sodium having a K/Na molar ratio range from 99/1 to 20/80 resulting in potassium concentrations ranging from 13.9 % by weight to 1.2 % by weight and sodium concentrations ranging from 0.04 % by weight to 6.6 % by weight.

The process according to the present invention may also lead to purge solutions having a K/Na molar ratio range from 90/10 to 20/80 resulting in potassium concentrations ranging from 12,6 % by weight to 1,2 % by weight and sodium concentrations ranging from 0,35 % by weight to 6,6 % by weight.

The process according to the present invention may also lead to purge solutions having a K/Na molar ratio range from 70/30 to 50/50 resulting in potassium concentrations ranging from 9,8 % by weight to 3 % by weight and sodium concentrations ranging from 1,06 % by weight to 4,1 % by weight.

Due to the potassium, nitrogen, sulphur and sodium content, the purge solution so obtained is suitable as a liquid fertilizer (C.C. Mitchel and A.E. Hiltbold, Journal of Plant Nutrition, 17(12), 2119-2134, 1994). Furthermore, sodium is considered being a functional nutrient for plants, i.e. an element which promotes maximal biomass yield and/or which reduces the requirement (critical level) of an essential element (Subbarao et al., Critical Reviews in Plant Sciences, 22(5):391-416 (2003)).

Therefore, the present invention also relates to an aqueous solution comprising 2 to 6 % by weight methionine, 4 to 8 % by weight methionylmethionine, 1 to 1.7 % by weight formate, 0.3 to 0.5 % by weight acetate and 13.9 % by weight to 1.2 % by weight potassium in form of potassium salts and 0.04 % by weight to 6.6 % by weight sodium in form of sodium salts. The solution may also have potassium concentrations ranging from 12,6 % by weight to 1,2 % by weight and sodium concentrations ranging from 0,35 % by weight to 6,6 % by weight. The solution according to the present invention may alternatively have potassium concentrations ranging from 9,8 % by weight to 3 % by weight and sodium concentrations ranging from 1,06 % by weight to 4,1 % by weight.

Due to its composition, the aqueous solution according to the present invention may be used as fertilizer or fertilizer additive.

**Brief description of the Figures**

**Figure 1** shows a scheme of the hydantoin hydrolysis circulation.

**Figure 2** shows the solubility curves of $KHCO_3$, $NaHCO_3$ and methionine in water. The measured values stem from the following sources.
Solubility of $KHCO_3$:

Seidell, 1940: Seidel, A.; Solubility Data Ser., 1940.
Gmelin: Gmelins Handbuch, 22, 1938.
Takahashi, 1927: Takahashi, G.; Bull. Imp. Seric. Stn. Tokyo 29, 1927, 165.

Solubility of $NaHCO_3$:

Trypuc & Kielkowska, 1998: Trypuc, M.; Kielkowska, U.; J. Chem. Eng. Data 43 (2), 1998, 201-204. Fedotev, 1904: Fedotev, P. P., Z. Phys. Chem. 49, 1904, 168.

Solubility of Methionine:

Fuchs et al., 2006: Fuchs et al., 2006, Ind. Eng. Chem. Res., 45, 6578-6584.
Lab Exp: Results of own laboratory experiments (in accordance to Fuchs et al., 2006).

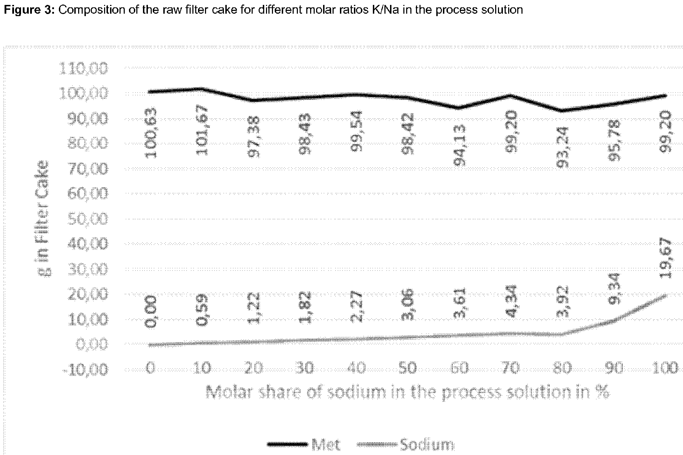

**Figure 3** shows the composition of the raw filter cake for different molar ratios K/Na in the process solution (Example 1).

**Experimental Part**

In the carbonation reaction the potassium salt of methionine (K-Met) accompanied by 0.5 equivalents of potassium carbonate is reacted with $CO_2$. $KHCO_3$ is formed and methionine (H-Met) is precipitated (Equations 1 and 2).

$$\text{K-Met} + CO_2 + H_2O \rightarrow \text{H-Met}_{(s)} + KHCO_3 \qquad \text{(Equation 1)}$$

$$K_2CO_3 + CO_2 + H_2O \rightarrow 2\, KHCO_3 \qquad \text{(Equation 2)}$$

Due to the significantly lower solubility of $NaHCO_3$ compared to $KHCO_3$ (Figure 2) the molar ratio of K/Na has to be carefully tuned in order to avoid undesired precipitation of $NaHCO_3$ crystals in the carbonation reactor.

Thus, a series of experiments was carried out in the laboratory to investigate the carbonation reaction with synthetic process solutions for different molar ratios K/Na. The overall alkaline concentration (sum parameter of K and Na) is maintained constant meaning that for a molar ratio K/Na = 70/30 compared to K/Na = 100/0 the potassium concentration is lowered by 30 % and replaced equimolar by sodium. The results of this study are summarized in Figure 3.

The methionine content in the filter cake can be considered being constant for molar ratios K/Na 100/0 to 0/100. The Na-content in the filter cake is slightly increasing for molar ratios K/Na 100/0 to 20/80 up to 3,92 g which can be explained by process solution being attached to and incorporated into the filter cake. However, for molar ratios K/Na 10/90 and 0/100 significantly higher Na content 9.34 - 19.67 g) was observed in the filter cake indicating that $NaHCO_3$ is also partially precipitated.

Thus, it is possible to run the carbonation reaction up to molar ratios K/Na 20/80 without coprecipitations of sodium hydrogencarbonate.

**Example 1**

Experimental procedure for the preparation of a process solution with a molar ratio K/Na of 90/10

A 1000 ml beaker was placed in an ice bath and KOH (40% in water, 354.2 g, 2.53 mol), NaOH (40% in water, 11.2 g, 0.28 mol), D,L-methionine (159.7 g, 1.07 mol), DL-methionyl-DL-methionine (29.9 g, 0.11 mol) and formic acid (50% in water, 41.4 g, 0.45 mol) were dissolved in 200 ml DI-water and stirred for 45 min. The solution was then warmed to 20 °C, transferred to a 1000 ml volumetric flask and filled up with DI-water to 1000 ml total volume.

1100 g of the solution was then transferred to a 2 l Büchi laboratory autoclave and 0.32 g of Defoamer EG2007 were added. Cooling of the autoclave to 30 °C was realized via cooling jacket and cryostat. Mechanical stirring was initiated and the autoclave was pressurized with $CO_2$ gas to 2 barg. The $CO_2$ gas feedstream was adjusted to maintain 2 barg until pH 8 was reached and temperature was maintained at 30 °C. Pressure was released from the autoclave and the suspension was transferred to a vacuum filter frit and sucked dry for 15 min at 940 mbar absolute pressure. The filtrate was collected in a 100 ml glas bottle and diluted with DI-water to avoid precipitation of solids afterwards and balanced subsequently. The filter cake was transferred to 3000 ml beaker via spatula and residual solids in the frit were rinsed into the beaker via DI-water. Additional DI-water was added until the solids have been dissolved. The solution was then transferred to a 3 l glass bottle and balanced. Moreover, the residual solids in the autoclave were also rinsed with DI-water collected in a glass bottle as so-called "Holdup" and balanced.

The synthetic process solution, the filter cake dissolved in DI-water, the filtrate and the "Holdup" were analysed for their K and Na content via IPC-OES, Met- & Met-Met via HPLC and formate via ion chromatography and the results are summarized in Table 1.

**Table 1:** Carbonation reaction of synthetic process solution with molar ratio K/Na of 90/10.

|  | Quantity g | % Met | g Met | % Met-Met | g Met-Met | % K | g K | % Na | g Na |
|---|---|---|---|---|---|---|---|---|---|
| Start | 1099,85 | 14,14 | 155,52 | 2,58 | 28,38 | 8,2 | 90,19 | 0,59 | 6,489 |
| Filtrate | 1001,1 | 4,14 | 41,45 | 2,25 | 22,52 | 7,1 | 71,08 | 0,51 | 5,106 |
| Cake | 2930,1 | 3,47 | 101,67 | 0,120 | 3,52 | 0,3 | 8,79 | 0,02 | 0,586 |
| Holdup | 498,8 | 1,88 | 9,38 | 0,25 | 1,25 | 0,75 | 3,74 | 0,05 | 0,249 |
| m F+C+H [g] |  |  | 152,50 |  | 27,29 |  | 83,61 |  | 5,94 |
| Accuracy [%] |  |  | 98,06 |  | 96,16 |  | 92,71 |  | 91,55 |

**Claims**

1. A process for preparing methionine, comprising the alkaline hydrolysis of 5-(2-methylmercaptoethyl)-hydantoin (methionine hydantoin) in an aqueous process solution comprising alkali metal hydroxide and/or alkali metal carbonate and/or alkali metal hydrogencarbonate to form methionine alkali metal salt, precipitation of methionine by means of carbon dioxide, separation of the precipitated methionine from the process solution, concentration of the process solution and recycling of the process solution in the alkaline hydrolysis of methionine hydantoin once more forwarded to the process solution, wherein the alkali metal cations in the process solution are potassium and sodium having a K/Na molar ratio range from 99/1 to 20/80.

2. The process as claimed in claim 1, wherein the K/Na molar ratio in the process solution ranges from 90/10 to 20/80.

3. The process as claimed in claim 1 or 2, wherein the K/Na molar ratio in the process solution ranges from 70/30 to 50/50.

4. The process as claimed in any of claims 1 to 3, wherein the precipitated methionine is recrystallized from an aqueous solution, filtered off and dried and wherein the crystalline methionine so obtained has a purity greater than 99 % and a potassium content lower than 0.3 % and a sodium content lower than 0.1 %

5. The process as claimed in claim 4, wherein the potassium content in the crystalline methionine ranges from 0.05 - 0.3 % and the sodium content ranges from 0.01 - 0.1 %.

6. The process as claimed in any of claims 1 to 6, wherein a part of the process solution, the purge solution, is continuously removed from the process and exchanged by fresh alkali metal hydroxide solution.

7. The process as claimed in claim 6, wherein the purge solution comprises 2 to 6 % by weight methionine, 4 to 8 % by weight methionylmethionine, 1 to 1.7 % by weight formate, 0.3 to 0.5 % by weight acetate and 6 to 14 % by weight alkali metal in the form of potassium and sodium salts, potassium and sodium having a K/Na molar ratio range from 99/1 to 20/80 resulting in potassium concentrations ranging from 13.9 % by weight to 1.2 % by weight and sodium concentrations ranging from 0.04 % by weight to 6.6 % by weight.

8. The process as claimed in claim 7, wherein the purge solution has a K/Na molar ratio range from 90/10 to 20/80 resulting in potassium concentrations ranging from 12,6 % by weight to 1,2 % by weight and sodium concentrations ranging from 0,35 % by weight to 6,6 % by weight.

9. The process as claimed in claim 8 wherein the purge solution has a K/Na molar ratio range from 70/30 to 50/50 resulting potassium concentrations ranging from 9,8 % by weight to 3 % by weight and sodium concentrations ranging from 1,06 % by weight to 4,1 % by weight.

10. Aqueous solution comprising 2 to 6 % by weight methionine, 4 to 8 % by weight methionylmethionine, 1 to 1.7 % by weight formate, 0.3 to 0.5 % by weight acetate and 13.9 % by weight to 1.2 % by weight potassium in form of potassium salts and 0.04 % by weight to 6.6 % by weight sodium in form of sodium salts.

11. Aqueous solution as claimed in claim 10 having potassium concentrations ranging from 12,6 % by weight to 1,2 % by weight and sodium concentrations ranging from 0,35 % by weight to 6,6 % by weight.

12. Aqueous solution as claimed in claim 11 having potassium concentrations ranging from 9,8 % by weight to 3 % by weight and sodium concentrations ranging from 1,06 % by weight to 4,1 % by weight.

13. Use of the aqueous solution as claimed in any of claims 10 to 12 as fertilizer or fertilizer additive.

**Figure 1**

Figure 2: Solubility curves of KHCO₃, NaHCO₃ and methionine in water

**Figure 3:** Composition of the raw filter cake for different molar ratios K/Na in the process solution

Europäisches
Patentamt

European
Patent Office

Office européen
des brevets

# EUROPEAN SEARCH REPORT

Application Number

EP 17 18 2515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2016/170252 A1 (ADISSEO FRANCE SAS [FR]) 27 October 2016 (2016-10-27) <br> * page 5 - page 6; examples 1-3 * <br> * claim 1 * | 1-9 | INV. <br> C07C319/20 <br> C07C319/28 <br> C07C323/58 |
| A | US 4 391 988 A (SPINDLER MANFRED [DE] ET AL) 5 July 1983 (1983-07-05) <br> * column 2 - column 3; examples 1-4 * <br> * claim 1 * | 1-9 | |
| A | EP 0 839 804 A2 (SUMITOMO CHEMICAL CO [JP]) 6 May 1998 (1998-05-06) <br> * claim 1 * | 1-9 | |
| A | EP 2 133 328 A2 (SUMITOMO CHEMICAL CO [JP]) 16 December 2009 (2009-12-16) <br> * claim 1 * | 1-9 | |
| A | US 4 259 525 A (SCHAAF KURT H) 31 March 1981 (1981-03-31) <br> * abstract * | 1-9 | |
| A | US 4 272 631 A (SCHAAF KURT H ET AL) 9 June 1981 (1981-06-09) <br> * abstract * | 1-9 | TECHNICAL FIELDS SEARCHED (IPC) <br><br> C07C |
| A,D | MITCHELL C C ET AL: "Evaluation of a liquid potassium bicarbonate/amino acid co-product as a source of potassium, nitrogen, and sulfur", JOURNAL OF PLANT NUTRI, MARCEL DEKKER, NEW YORK, NY, US, vol. 17, no. 12, 1 November 1994 (1994-11-01), pages 2119-2134, XP008125142, ISSN: 0190-4167, DOI: 10.1080/01904169409364869 <br> * the whole document * | 1-9 | |

-/--

~~The present search report has been drawn up for all claims~~

| Place of search | Date of completion of the search | Examiner |
|---|---|---|
| Munich | 9 October 2017 | Bissmire, Stewart |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 18 2515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | G. V. SUBBARAO ET AL: "Sodium-A Functional Plant Nutrient", CRITICAL REVIEWS IN PLANT SCIENCES., vol. 22, no. 5, 1 September 2003 (2003-09-01), pages 391-416, XP055413499, US ISSN: 0735-2689, DOI: 10.1080/07352680390243495 * the whole document * ----- | 1-9 | |

TECHNICAL FIELDS
SEARCHED     (IPC)

~~The present search report has been drawn up for all claims~~

| Place of search | Date of completion of the search | Examiner |
|---|---|---|
| Munich | 9 October 2017 | Bissmire, Stewart |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 17 18 2515

The Search Division considers that the present European patent application does not comply with the
requirements of unity of invention and relates to several inventions or groups of inventions, namely:

    1. claims: 1-9

        A process for the preparation of methionine.
                        ---

    2. claims: 10-13

        An aqueous solution containing methionine,
        methionylmethionine, formate, acetate, potassium salt and
        sodium salts for use as a fertilizer or fertilizer additive.
                        ---

## EP 3 431 465 A1

### ANNEX TO THE EUROPEAN SEARCH REPORT
### ON EUROPEAN PATENT APPLICATION NO.

EP 17 18 2515

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2017

| Patent document cited in search report | | Publication date | Patent family member(s) | | Publication date |
|---|---|---|---|---|---|
| WO 2016170252 | A1 | 27-10-2016 | CN | 107531621 A | 02-01-2018 |
| | | | FR | 3035400 A1 | 28-10-2016 |
| | | | KR | 20170140167 A | 20-12-2017 |
| | | | SG | 11201708366Q A | 29-11-2017 |
| | | | TW | 201704207 A | 01-02-2017 |
| | | | WO | 2016170252 A1 | 27-10-2016 |
| US 4391988 | A | 05-07-1983 | BE | 892113 A | 11-08-1982 |
| | | | DE | 3104997 A1 | 26-08-1982 |
| | | | ES | 8302643 A1 | 16-01-1983 |
| | | | FR | 2499563 A1 | 13-08-1982 |
| | | | JP | S57150657 A | 17-09-1982 |
| | | | US | 4391988 A | 05-07-1983 |
| EP 0839804 | A2 | 06-05-1998 | BR | 9705174 A | 20-07-1999 |
| | | | CN | 1181378 A | 13-05-1998 |
| | | | DE | 69709526 D1 | 14-02-2002 |
| | | | DE | 69709526 T2 | 01-08-2002 |
| | | | EP | 0839804 A2 | 06-05-1998 |
| | | | ES | 2166036 T3 | 01-04-2002 |
| | | | RU | 2208943 C2 | 27-07-2003 |
| | | | US | 5945563 A | 31-08-1999 |
| EP 2133328 | A2 | 16-12-2009 | CN | 101602700 A | 16-12-2009 |
| | | | EP | 2133328 A2 | 16-12-2009 |
| | | | JP | 2009292796 A | 17-12-2009 |
| | | | SG | 158027 A1 | 29-01-2010 |
| | | | US | 2010004486 A1 | 07-01-2010 |
| US 4259525 | A | 31-03-1981 | NONE | | |
| US 4272631 | A | 09-06-1981 | NONE | | |

EPO FORM P0459

For more details about this annex : see Official Journal of the European Patent Office, No. 12/82

14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5770769 A
- WO 2013030068 A2
- EP 1564208 A1
- WO 2016170252 A1

### Non-patent literature cited in the description

- **C.C. MITCHEL ; A.E. HILTBOLD.** *Journal of Plant Nutrition,* 1994, vol. 17 (12), 2119-2134
- **SUBBARAO et al.** *Critical Reviews in Plant Sciences,* 2003, vol. 22 (5), 391-416
- **SEIDEL, A.** *Solubility Data Ser.,* 1940
- Gmelins Handbuch. 1938, 22
- **TAKAHASHI, G.** *Bull. Imp. Seric. Stn.,* 1927, vol. 29, 165
- **TRYPUC, M. ; KIELKOWSKA, U.** *J. Chem. Eng. Data,* 1998, vol. 43 (2), 201-204
- **FEDOTEV, P. P., Z.** *Phys. Chem.,* 1904, vol. 49, 168
- **FUCHS et al.** *Ind. Eng. Chem. Res.,* 2006, vol. 45, 6578-6584
- **FUCHS et al.** *Lab Exp: Results of own laboratory experiments,* 2006